# EUROPÄISCHE PATENTSCHRIFT

(11) **EP 0 310 669 B1**
(45) Veröffentlichungstag und Bekanntmachung des Hinweises auf die Patenterteilung: **03.11.1993**
(21) Anmeldenummer: 88901114.4
(22) Anmeldetag: 05.01.1988
(51) Int. Cl.: A61B 17/42, A61M 1/00

(54) **Gynäkologische Absaugtülle**
Gynaecological suction nozzle
Canule utérine à aspiration

(30) Priorität: 08.01.1987 SU 4176523
(43) Veröffentlichungstag der Anmeldung: 12.04.1989
(73) Patentinhaber: Gainutdinova, Raisa Vladimirovna, Kazan 420049 (SU)
(72) Erfinder: GAINUTDINOVA, Raisa Vladimirovna, Kazan, 420049 (SU); MANUILOVA, Irina Alexandrovna, Moscow, 121293 (SU); PETROVA, Vera Mitrofanovna, Kazan, 420033 (SU); ZHUKOVSKY, Yakov Grigorievich, Moscow, 117311 (SU)
(74) Vertreter: Nix, Frank Arnold, Dr.
(86) Internationale Anmeldenummer: SU8800002
(87) Internationale Veröffentlichungsnummer: WO8804913

(56) Entgegenhaltungen:
- DE-A- 3 033 459
- FR-A- 2 238 464
- FR-A- 2 240 026
- US-A- 1 879 249
- US-A- 3 965 901
- US-A- 4 451 257
- US-A- 4 504 266

## Beschreibung

Die vorliegende Erfindung bezieht sich auf Einrichtungen zur Schwangerschaftsunterbrechung im frühen Stadium, insbesondere auf eine gynäkologische Absaugtülle.

Die Erfindung kann in der Gynäkologie zur Schwangerschaftsunterbrechung im frühen Stadium sowie zum Absaugen des Inhaltes der Gebärmutterhöhle bei verschiedenen pathologischen Änderungen der Gebärmutter, beispielsweise bei hyperplastischen Endometriumänderungen, bei Krebsverdacht, bei Unfruchtbarkeit, bei Polypen am Gebärmutterhals (an der Cervix) angewendet werden.

Die vorliegende Erfindung gestattet es, eine vollständigere Absaugung des Inhaltes der Gebärmutterhöhle schmerzfrei vorzunehmen.

Bekannt sind gynäkologische Absaugtüllen zweier Typen: Absaugtüllen mit schabender Wirkung und reine Absaugtüllen.

Im ersten Fall stellt die Absaugtülle ein zylindrisches Rohr mit geschlossenem distalem Ende und zwei Öffnungen in den Seitenwänden dar. Über einer der Öffnungen ist ein spitzer dreieckiger Vorsprung vorhanden, der die schabende Wirkung sicherstellt.

Die bekannte Einrichtung verletzt beim Ausschaben der Wände der Gebärmutterhöhle die Nervenenden, die sich in den Gebärmutterwänden befinden, was spätere Komplikationen nach sich zieht.

Im zweiten Fall stellt die Absaugtülle ebenfalls ein zylindrisches Rohr mit geschlossenem bzw. offenem distalen Ende und Öffnungen in den Seitenwänden dar, allerdings ohne den spitzen Vorsprung. Diese Absaugtüllen sind zur Abführung des Inhaltes der Gebärmutterhöhle ausschließlich durch saugende Wirkung bestimmt.

Allerdings werden die an den Seitenwänden der Absaugtülle vorhandenen Öffnungen durch Schleimhautstückchen zugesetzt, die die Absaugtülle verstopfen, so daß die Wirkung aufhört. Die erneute Einführung der Absaugtülle kann eine Infizierung der Gebärmutterhöhle bewirken.

Aus der US-PS 3 965 901 ist eine Absaugtülle bekannt, die ein flexibles zylindrisches Rohr mit offenem Stirnende darstellt. Am distalen Ende desselben ist in der Seitenwand eine T-förmige Nut vorhanden, die mit dem Rohrinnenraum in Verbindung steht. Das proximale Ende ist zur Verbindung mit einer Vakuumquelle vorgesehen.

Die bekannte Einrichtung ist dadurch gekennzeichnet, daß beim Einführen der Absaugtülle in die Gebärmutterhöhle und Erzeugen eines Unterdrucks die durch Schwangerschaft bzw. pathologische Prozesse veränderte Schleimhaut der Gebärmutterhöhle beginnt, durch die in der Absaugtülle vorhandenen Öffnungen in deren Innenraum und dann in einen Sammelbehälter für die abgesaugte Substanz zu gelangen.

Jedoch kann es beim Absaugen mit Hilfe der bekannten Absaugtülle zum Zusetzen und Verstopfen der Nut durch größere Schleimhautstücke kommen, so daß die Wirkung aufhört und ein Herausziehen der Absaugtülle aus der Gebärmutterhöhle und Reinigen der Nut notwendig wird. Die erneute Einführung der Absaugtülle in die Gebärmutterhöhle kann eine Infizierung der Gebärmutter mit späteren Komplikationen zur Folge haben.

Bei der bekannten Einrichtung kann es bei gewissen Werten des Unterdrucks beim Absaugen zu einer Berührung der Nut der Absaugtülle mit der Schleimhaut der Gebärmutterhöhle kommen, wodurch Schmerzempfindungen eintreten können, die durch eine recht große Berührungsfläche bedingt sind.

Ähnliches gilt für eine aus der Druckschrift FR-A-2 238 464 bekannte Absaugtülle mit geschlossenem und abgerundetem distalen Ende, die in der Nähe dieses Endes in der Seitenwand eine größere schabende Öffnung mit scharfen Rändern und in etwas größerer Entfernung vom distalen Ende und auf dem Umfang gegenüberliegend eine halbrunde Öffnung aufweist. Beide Öffnungen dienen zur Abführung des Gebärmutterinhalts.

Aus der US-Patentschrift 1 879 249 ist ein flexibles Darmspülungsrohr bekannt, das eine stirnseitige Öffnung verhältnismäßig kleinen Durchmessers aufweist, welche über einen sich verengenden Schlitz in eine Seitenwandöffnung in der Nähe des Stirnendes übergeht. Ein solches Darmspülungsrohr ist nur für die Abführung von Flüssigkeit und nicht als gynäkologische Absaugtülle geeignet.

Der Erfindung liegt die Aufgabe zugrunde, eine gynäkologische Absaugtülle zu schaffen, mit der eine gravidäre oder pathologisch veränderte Schleimhaut der Gebärmutterhöhle ohne Unterbrechungen des Operationsvorganges qualitativ besser und bei hoher Sicherheit gegen Infizierung entfernt werden kann.

Die gestellte Aufgabe ist dadurch gelöst, daß bei einer gynäkologischen Absaugtülle in Form eines flexiblen zylindrischen Rohres mit einer in dessen Wand ausgebildeten Nut, die mit dem Innenraum des Rohres in Verbindung steht und sich an dessen distalem Ende befindet, während sein proximales Ende zur Verbindung mit einer Vakuumquelle vorgesehen ist, erfindungsgemäß die Nut zur Stirnseite des distalen Rohrendes offen ausgeführt ist und das Verhältnis der Nutbreite zur Nutlänge von etwa 1:6 bis etwa 1:10 beträgt, während die Nutbreite von etwa 1/3 bis etwa 1/2 des Außendurchmessers des Rohres ausmacht.

Eine solche Ausführung der Absaugtülle gewährleistet eine maximale Berührungsfläche der Nut mit den Wänden der Gebärmutterhöhle, was das Absaugen unter vollständiger Entfernung der Schleimhaut vom Boden sowie von den Seitenwänden der Gebärmutter durchzuführen erlaubt.

Darüber hinaus gestattet es diese Nutausbildung, die Absaugtülle durch den Halskanal der Gebärmutter ohne vorherige Erweiterung desselben einzuführen, weil die elastischen Wände des Halskanals das distale Ende der Tülle zusammendrücken und den Kreisumfang der stirnseitigen Öffnung um die Nutbreite verringern, wobei dadurch auch der Außendurchmesser der stirnseitigen Öffnung vermindert wird.

Die Nutabmessungen gewährleisten eine vollständige Entfernung der Schleimhaut ohne Schmerzempfindungen bei der Patientin bei einem vorgegebenen Unterdruck.

Eine Vergrößerung der Nutabmessungen kann zu einem Schmerzsyndrom führen und eine Verkleinerung derselben führt zum Zusetzen der Öffnungen durch einzelne Schleimhautstückchen.

Zweckmäßigerweise geht die Nut an ihrem offenen Ende ausgerundet in die Stirnseite des distalen Rohrendes über. Diese Ausbildung der Nutränder schließt Verletzungen durch die Absaugtülle aus.

### Kurze Beschreibung der Zeichnungen

Im folgenden wird die Erfindung durch ein Beispiel ihrer konkreten Ausführung unter Bezugnahme auf beiliegende Zeichnungen näher erläutert; in den Zeichnungen zeigt:
Fig. 1 gynäkologische Absaugtülle gemäß der Erfindung, Gesamtansicht;
Fig. 2 die in die Gebärmutterhöhle durch den Gebärmutterhalskanal eingeführte Absaugtülle gemäß der Erfindung;
Fig. 3 den Schnitt nach der Linie III-III von Fig. 2 im Augenblick der Beendigung der Operation mit bedingt nicht geschnitten dargestelltem Saugrohr.

### Beste Ausführungsform der Erfindung

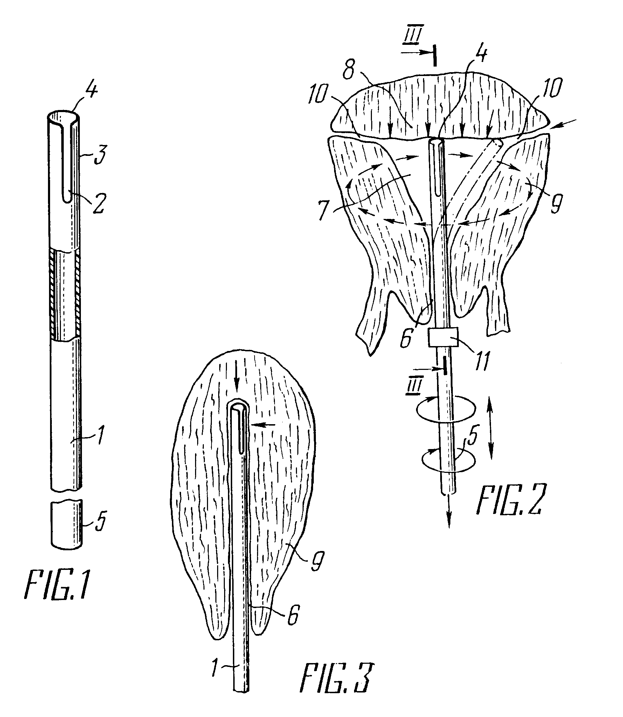

Die gynäkologische Absaugtülle ist als flexibles zylindrisches Hohlrohr 1 (Fig. 1) ausgebildet. An seiner Wand ist eine Nut 2 vorhanden, die mit dem Innenraum des Rohres 1 in Verbindung steht. Die Nut 2 befindet sich am distalen Ende 3 des Rohres 1.

Die Nut 2 ist an der Stirnseite 4 des distalen Endes 3 des Rohres 1 offen ausgeführt und mit dieser Stirnseite 4 verbunden.

Dank der Vereinigung der Nut 2 mit der stirnseitigen Öffnung ist eine vollständigere Entfernung der gravidären Schleimhaut aus den Gebärmutterecken und die Beseitigung des Schmerzsyndroms gewährleistet.

Das proximale Ende 5 des Rohres 1 ist zur Verbindung mit einer Vakuumquekle vorgesehen.

In Fig. 2 ist die Absaugtülle im Gange einer Operation dargestellt.

Die Absaugtülle wird ohne Erweiterung des Halskanals 6 in eine Gebärmutterhöhle 7 bis zur Berührung der Stirnseite 4 mit dem Gebärmutterboden 8, den Gebärmutterseitenwänden 9 und dem Bereich der Gebärmutterecken 10 eingeführt.

Am Rohr 1 ist ein Begrenzer 11 vorgesehen, dessen Lage durch die Tiefe der Gebärmutterhöhle 7 bestimmt ist. Die Tiefe der Gebärmutterhöhle 7 wird durch vorherige Sondierung ermittelt. Der Begrenzer 11 wird am Rohr 1 in einem Abstand von dessen distalem Ende 3 angeordnet, welcher der Tiefe der Gebärmutterhöhle 7 gleich ist, was seine Einführung nur bis auf diese Tiefe ermöglicht. Dadurch kann eine Verletzung der Gebärmutter vermieden werden.

In Fig. 3 ist der Augenblick der Operationsbeendigung dargestellt. Die Absaugtülle ist von den Gebärmutterwänden 9 und den Wänden des Halskanals 6 dicht umfaßt.

Die gynäkologische Absaugtülle wird folgenderweise eingesetzt.

Die Absaugtülle (Fig. 2) wird ohne vorherige Erweiterung des Halskanals 6 in die Gebärmutterhöhle 7 bis zur Berührung der Stirnseite 4 mit dem Gebärmutterboden 8 eingeführt. Das proximale Ende 5 der Absaugtülle wird an eine Vakuumquelle angeschlossen, ein unter dem atmosphärischen liegender Druck wird zugeführt, und ferner wird die Absaugtülle um ihre Achse unter Neigen bald nach der einen, bald nach der anderen Gebärmutterecke 10 bei gleichzeitiger hin- und hergehender Bewegung gedreht.

Während der Operation erwärmt sich der in der Gebärmutterhöhle 7 befindliche Teil der Absaugtülle. Dadurch wird er noch elastischer und an die Seitenwände 9 der Gebärmutter abgelenkt, was zu einer qualitativ besseren Durchführung der Operation beiträgt.

Das Verhältnis der Nutbreite zur Nutlänge beträgt für Frauen, die noch nicht geboren haben, etwa 1:6, für Frauen mit früheren Geburten etwa 1:10. Dabei beträgt das Verhältnis der Nutbreite zum Nutaußendurchmesser für erstgebärende Frauen vorwiegend 1:3, für Frauen mit früheren Geburten aber vorwiegend 1:2.

Somit kann dank der Ausführung der gynäkologischen Absaugtülle auf die im vorstehenden beschriebene Weise eine Verstopfung der Nut durch Teilchen der Gebärmutterschleimhaut vermieden werden, was die Dauer der Operation zu verkürzen und die Infizierung der Gebärmutterhöhle zu vermeiden erlaubt, während die Wahl optimaler Verhältnisse von Nutbreite und -länge sowie von Außendurchmesser der Absaugtülle es gestattet, ein Schmerzsyndrom bei der Patientin zu vermeiden.

### Gewerbliche Verwertbarkeit

Die vorliegende Erfindung kann in der Gynäkologie zur Schwangerschaftsunterbrechung im frühen Stadium und zur Biopsie des Endometriums angewendet werden.

## Patentansprüche

1. Gynäkologische Absaugtülle in Form eines flexiblen zylindrischen Rohres (1) mit einer in dessen Wand ausgebildeten Nut (2), die mit dem Innenraum dieses Rohres (1) in Verbindung steht und sich an dessen distalem Ende (3) befindet, während das proximale Ende (5) des Rohres (1) zur Verbindung mit einer Vakuumquelle vorgesehen ist,
dadurch gekennzeichnet, daß die Nut (2) zur Stirnseite (4) des distalen Endes (3) des Rohres (1) offen ausgeführt ist
und daß das Verhältnis der Breite der Nut (2) zur Länge derselben von etwa 1:6 bis etwa 1:10 beträgt, während die Breite der Nut (2) von etwa 1/3 bis etwa 1/2 des Außendurchmessers des Rohres (1) ausmacht.

## Claims

1. A gynaecological suction nozzle in the form of a flexible cylindrical tube (1) with a groove (2) formed in its wall, which groove (2) is connected to the interior space of this tube (1) and is located at the distal end (3) thereof, whereas the proximal end (5) of the tube (1) is provided for connection to a vacuum source,
characterised in that the groove (2) is open on the front side (4) of the distal end (3) of the tube (1),
and in that the ratio of the width of the groove (2) to its length is approximately 1 : 6 to 1 : 10, whereas the width of the groove (2) amounts to approximately 1/3 to 1/2 of the external diameter of the tube (1).

## Revendications

1. Canule utérine à aspiration, se présentant sous la forme d'un tube cylindrique (1) flexible avec une rainure (2) réalisée dans sa paroi, reliée à l'espace intérieur de ce tube (1) et se trouvant à son extrémité distale (3), tandis que l'extrémité proximale (5) du tube est prévue pour assurer la liaison avec une source de vide, caractérisée en ce que la rainure (2) est ouverte vers le côté frontal (4) de l'extrémité distale (3) du tube (1) et que le rapport entre la largeur de la rainure (2) et sa longueur est compris dans la plage allant d'à peu près 1/6 à à peu près 1/10 tandis que la largeur de la rainure (2) fait d'à peu près 1/3 à à peu près 1/2 du diamètre extérieur du tube (1).
